(19) Europäisches Patentamt
European Patent Office
Office européen des brevets

(11) **EP 2 836 834 B1**

(12) **EUROPEAN PATENT SPECIFICATION**

(45) Date of publication and mention
of the grant of the patent:
**30.03.2016 Bulletin 2016/13**

(21) Application number: **13718030.3**

(22) Date of filing: **15.04.2013**

(51) Int Cl.:
*G01N 33/543* (2006.01)     *G01N 33/58* (2006.01)
*G01N 33/548* (2006.01)

(86) International application number:
**PCT/GB2013/000169**

(87) International publication number:
**WO 2013/153355 (17.10.2013 Gazette 2013/42)**

(54) **AN ASSAY LABEL**

TESTETIKETT

MARQUE DE DOSAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.04.2012 GB 201206530
13.04.2012 US 201261623729 P**

(43) Date of publication of application:
**18.02.2015 Bulletin 2015/08**

(73) Proprietor: **Vivacta Limited
Kent Science Park
Sittingbourne
Kent ME9 8GU (GB)**

(72) Inventors:
• **ROSS, Steven
Ashford
Kent TN26 2HS (GB)**
• **MCGETTRICK, Aileen
Herne Bay
Kent CT6 6HW (GB)**
• **RICHARDS, Julie
Faversham
Kent ME13 TTE (GB)**

• **DWYER, Timothy
Sittingbourne
Kent ME10 4EL (GB)**
• **CAMERON, Helen
Wokingham
Berkshire RG41 1HR (GB)**
• **CARTER, Timothy
Minster-on-Sea
Sheerness Kent ME12 2LH (GB)**

(74) Representative: **Gillard, Richard Edward
Elkington and Fife LLP
Thavies Inn House
3-4 Holborn Circus
London EC1N 2HA (GB)**

(56) References cited:
**WO-A1-93/15117          WO-A1-2011/080524
WO-A2-2005/121163     KR-A- 20110 128 606
US-A1- 2003 134 333**

• **S A ROSS ET AL: "Whole blood cardiac troponin
I assay in 10 minutes from sample collection",
OAKRIDGE CONFERENCE, 19 December 2012
(2012-12-19), XP055068579,**

## Description

The present invention relates to an assay label and particularly an assay label based on a carbon particle for use in an immunoassay.

A number of assay systems use carbon particles as the detection conjugate (or label). One such example is a lateral-flow strip which uses carbon particles that are visualised by eye. Another example is the assay system described in WO 2004/090512, which employs a device having a pyroelectric/piezoelectric transducer for the detection of heat generated by illumination of the label during the assay. Carbon particles are well-suited to this system, since they are strong absorbers of electromagnetic radiation at a range of wavelengths, including the UV, visible and infra-red parts of the spectrum. Carbon particles are also not particularly mass dense, so they suspend well in the sample and do not overly sediment, which could cause interference in an assay system using a pyroelectric/piezoelectric transducer.

Particulate carbon is generally considered to have an "active" surface which can adsorb hydrophobic materials. For example, activated charcoal is used in filters for cooker hoods, and carbon particles are used to purify chemical reaction mixtures. In the latter, polymeric and/or hydrophobic impurities are commonly removed from a reaction vessel by adding activated charcoal, which adsorbs the impurities. The charcoal is then removed by filtration.

For this reason, carbon particles have been used in assays for some time, not as a label, but as a means of removing excess unwanted reagents. In particular, they have been used to remove excess radiolabelled reagents from competition immunoassays (e.g. insulin, folate, free T3, free T4). Such uses of carbon particles are described in US 3,442,819, US 4,028,465 and in N. Poznanski and W.J. Poznanski, Clin. Chem., 1969, 15, 908-918. In this technical application, a competition reaction is carried out in solution between a radiolabelled small molecule and an endogenous small molecule, where these compete for binding with a fixed amount of antibody. The small molecule fraction which does not bind to the antibody is then removed by adding carbon particles which have been pre-treated with dextran (or other macromolecules). The dextran allows small molecules to pass through and bind to the carbon, but not large molecules, so the carbon-dextran removes the unbound small molecule, but not the small molecule fraction bound to the antibody. The carbon-dextran is then filtered and the bound reagent is quantified by measuring the radioactivity of the remaining solution.

These documents teach the reader that carbon particles are likely to have a greater affinity for hydrophobic small molecules than they do for dextran. A common method for attaching small molecules to colloidal labels is firstly to attach the small molecule covalently to a larger carrier (such as a macromolecule) and then attach the conjugate to the label, i.e. by passive adsorption. However, if one prepares a covalent conjugate of dextran with a hydrophobic small molecule, one might expect binding of the conjugate to the carbon by attachment of the small molecule to the carbon. This is, therefore, unlikely to be a good way to prepare carbon conjugates of small molecules, because the small molecules would not be accessible.

There are examples in the literature describing the use of antibody-coated carbon particles as assay labels. For example, US 4,760,030 describes the passive adsorption of antibodies onto carbon particles. However, the particles in US 4,760,030 require stabilisation with amino acids in order to coat the antibody onto the particle. There is also some reference to the India-ink assay (also known as the Geck assay), which is an agglutination assay where carbon particles and antibody are mixed simultaneously, and agglutination takes place in the presence of an analyte. Again, stabilisers are required to form the carbon colloid.

US 5,252,496, US 5,559,041 and US 6,506,612 also describe antibody binding to carbon particles. These patents use Vulcan XC72 carbon particles, which must be stabilised in order to form a colloid in water. These patents describe the use of 2% dextran 9,400 as the stabilising agent. Example 7 in column 13 of US 5,252,496 describes immobilisation of a monoclonal antibody to the carbon. In summary, the carbon particles are homogenised in buffer with 2% dextran, the dextran being described as a "suspending adjuvant". After 2 h fluorescein isothiocyanate (FITC) is added and the suspension is incubated for 12 h, then washed several times before antibody being added, followed by further washing, and storage of the colloid in an appropriate storage buffer.

It is not entirely clear what the mechanism is by which the antibody is attached to the particle. Firstly, FITC has only one chemically reactive functional group and so is not a cross-linking agent, so there is no expected mechanism by which FITC could covalently link the antibody to the dextran. Secondly, isothiocyanates are normally used for reaction with amines (to yield thioureas), but can also react with alcohols (to give thiocarbamates) or be hydrolysed to amines. Given an incubation time of 12 h, the FITC will either react with the hydroxyl groups on the dextran or be hydrolysed. It appears that the proposed mechanism of binding is that the FITC somehow binds to the surface of the dextran carbon. Fluorescein would not be expected to bind to dextran, so this implies that the FITC is bound (*passively adsorbed*) onto the surface of the carbon itself. The isothiocyanate group would not then be available for binding to lysine groups (amines) on antibodies.

US 6,506,612 also describes methods whereby a binding agent is covalently bound through a cross-linker to a primary passively adsorbed layer on a carbon particle surface. Lines 35-60 of column 8 describe such covalent linking, where the passively adsorbed layer is always a protein. Thus, US6,506,612 teaches that dextrans can be used initially to stabilise carbon colloids, but that they will ultimately be displaced by either small molecules (such as FITC) or by protein molecules (such as BSA, avidin or antibodies), which will bind more strongly to the carbon than will the dextran. US 6,506,612 also describes a method for pre-treating an antibody with FITC before coating onto the carbon, the likely effect here is that adding FITC groups to the antibody makes it more hydrophobic and thus bind more strongly to the carbon.

US 5,529,901 and US 5,641,689 describe a method for selecting a type of carbon particle (SB4 from Degussa) that will form a colloid in water without the addition of stabilising agents, such as dextran, PEG, glycine etc. They also describe the selection of SB4 (and similar particles) for the direct conjugation of a binding component (e.g. an antibody) which binds to the analyte. These patents thus teach away from the use of dextran as a stabiliser. In fact, they describe the use of dextran to stabilise the particle as an unnecessary step, which can be removed by specifically using these particles.

The carbon particles that have typically been used in assays are conjugated using a passive adsorption method, see US 5,529,901 and US 5,641,689. This method is specific for certain types of carbon, in particular Spezial Schwartz 4 (SB4) from Degussa/Evonik (amorphous carbon particles having an average size of 100-200 nm), which form a stable colloid in water in the absence of any stabilisers. Alternatively, most other carbon particles require a stabiliser, such as a detergent or a macromolecule (e.g. dextran, PEG, glycine etc). The passive adsorption method is suitable for most antibody-based assays, but it should be noted that the majority of antibodies lose activity during passive adsorption, because the antibody is denatured as it binds to the surface (this is similar to what happens when antibody is passively adsorbed to the surface of microtitre plates). A useful rule-of-thumb is that approximately 10% of passively adsorbed antibody will be active, the remaining 90% being inactive, largely due to denaturation of the antibody.

For assays that require very high sensitivity, it is beneficial to have a higher loading of antibody on the carbon particles, which would require an alternative route to passive adsorption. Higher loading of antibody drives the thermodynamic equilibrium of the antibody-antigen interaction. Higher antibody loading per particle is preferred, rather than simply adding more particles, so that the system is not overloaded with particles (leading to non-specific binding). Additionally, passively adsorbed antibody is in close proximity to the surface of the carbon, which can make the antibody quite sterically hindered. Having antibody farther from the surface of the carbon may have benefits, in terms of reducing steric hindrance. Finally, passive adsorption of antibody onto the carbon leads to denaturation of the antibody, which can potentially promote non-specific binding.

In immunometric, (also known as sandwich or reagent-excess) immunoassays, as described in WO 2004/090512, an antibody (or similar reagent) is located on the sensor and another antibody is on the carbon particles. The carbon particles then bind to the sensor in the presence of analyte being detected. Both antibodies are present in a large excess.

In a competition assay, there is either (a) an antibody on the carbon particles and an analogue of the analyte (i.e. a small molecule) on the sensor or (b) an analogue (i.e. a small molecule) on the carbon particles and an antibody on the sensor. Binding takes place in absence of analyte, and is perturbed (reduced) in the presence of analyte.

The term "small molecule" is a term of the art in the field of immunoassays which is used to distinguish between molecules which can be measured in sandwich assays and those that cannot. To be measured in a sandwich assay, a molecule must be sufficiently large to have two or more distinguishable epitopes (antibody binding sites), so that two antibodies can bind to the molecule simultaneously, so that the molecule can be sandwiched between a capture antibody and a reporter (or labelled) antibody. If the molecule cannot form a sandwich, it falls into the class of "small" molecules. The molecular weight cut off is about 2,000-5,000.

When carrying out competition assays there is an additional requirement to have greater control over the levels of the various components in the system. For antibody bound to carbon, it is more difficult to control the amount of active antibody when using passive adsorption. In the alternative format for competition assays, it is the small molecule analogue which must be bound to the surface of the carbon particle. This is generally not possible with small molecules; either they will not adsorb to a surface because they are too small or, if they do adsorb to a surface, they are no longer recognised by antibodies. Small molecules are therefore generally conjugated to larger carriers, such as proteins, and the carrier then binds to the surface of interest, e.g. the carbon particle.

However, it has now been found that it is difficult to prepare small molecule-carbon conjugates through the traditional route of covalently attaching them to protein molecules (e.g. $\alpha_2$-macroglobulin, apoferritin, $\beta$-galactosidase, $\beta$-amylase, collagen (ovine), concanavalin A, keyhole limpet hemocyanin, myosin, urease, human thyroglobulin, porcine thyroglobulin and bovine thyroglobulin) and subsequently binding these proteins to the surface of the carbon particles. This may be because the small molecules are particularly hydrophobic (e.g. steroids, fluorescein, immunosuppressants). This may make the protein-small molecule conjugates too hydrophobic for coating onto carbon particles. The materials are either "sticky" (they give too much non-specific binding) or unstable over time in solution.

WO 2005/121163 describes a process for the isolation of one or more proteins from a protein solution, the process comprising the steps of: a) providing a protein solution comprising one or more specific proteins and having a preset pH and a preset ionic strength or conductivity, b) applying the protein solution to a packed bed or expanded bed column comprising an adsorbent, and c) obtaining one or more proteins from the column; wherein the protein solution has been supplemented with an alcohol.

KR 2011-0128606 describes magnetic nanoparticle-platinum nanoparticle-porous carbon complex and a method for manufacturing the same are provided to improve the activity of peroxidase by integrating platinum nanoparticles and magnetic nanoparticles in the pores of porous carbon. Magnetic nanoparticles are fixed to porous carbon to obtain a magnetic nanoparticle-porous carbon complex by immersing $Fe(NO_3)_3$ in ethanol and implementing a deposition process under an inert gas atmosphere. Platinum nanoparticles are fixed to the magnetic nanoparticle-porous carbon complex in order to obtain a magnetic nanoparticle-platinum nanoparticle-porous carbon complex by immersing $H_2PtCl_6 \cdot 6H_2O$ in 1 M sodium hydroxide/ethylene glycol solution and implement a deposition process under an inert gas atmosphere. The magnetic nanoparticle-platinum nanoparticle-porous carbon complex includes peroxidase activity.

Thus, there is a requirement for an improved method for conjugating antibodies and small molecules, as well as other potential binding agents, e.g. nucleic acids, to the surface of carbon particles in order to increase loading, control loading, reduce steric hindrance and improve stability, thereby improving the assay performance by lowering assay sensitivity and improving precision.

Accordingly, the present invention provides an assay label comprising an amorphous carbon particle, a functionalised dextran polymer passively adsorbed onto the surface of the carbon particle and a first member of a complementary binding pair covalently bonded to the functionalised dextran polymer.

Thus, the present invention provides a conjugated carbon particle employing dextran to attach the molecule of interest to the carbon particle.

The present invention will now be described with reference to the drawings, in which:

Fig. 1 shows a schematic representation of the chemical sensing device of WO 2004/090512 which is used with the present invention;
Fig. 2 shows a cartridge according to the present invention;
Fig. 3 shows a plot of carbon binding capacity assays using carbon conjugates 1 and 2;
Fig. 4 shows a graph of troponin I assays using carbon conjugates 1 and 2;
Fig. 5 shows a graph of BSA-dig(en)-FITC carbon assays over 29 days;
Fig. 6 shows a graph of reduction in chamber 3 binding of BSA-dig(en)-FITC carbon conjugate;
Fig. 7 shows a graph of chamber 3 binding after washing of BSA-dig(en)-FITC carbon conjugate;
Fig. 8 shows a graph of dextran-FITC-digoxin assays over 29 days; and

Fig. 9 shows a graph of chamber 3 binding of dextran-dig(en)-FITC conjugates over 29 days.

The assay label of the present invention is based on an amorphous carbon particle. Such particles are well known in the art and are also referred to as Carbon Black, Lamp Black, Furnace Black or thermal black). They are amorphous particles produced by the incomplete combustion of gaseous or liquid hydrocarbons. The CAS number for carbon black is 1333-86-4.

The size of the carbon particles will depend on the nature of the assay, but they typically have a particle size of 25-250 nm. Particle sizes for the carbon particles of the present invention represent the diameter of the particle at its widest point and may be measured by dynamic light scattering.

Amorphous carbon particles generally have a porous structure. The carbon particle used in the present invention preferably has a density of 0.8 to 3 g/mL.

The carbon particles of the present invention form stable colloids on suspension in an aqueous medium. Depending on the hydrophobicity of the carbon particles (some have more surface hydroxyl groups than other), some carbon particles will form a stable colloid in water and others require the presence of a stabiliser. The present invention preferably uses carbon particles which form a stable colloid in water without the presence of a stabiliser (indeed, without the presence of any other substance). A simple test may be applied to determine whether or not a stable colloid is formed: suspend 1% w/v of the amorphous carbon particles in 1 mL of demineralised water and sonicate for 30 s to form a colloid; measure the optical density of the colloid using a visible spectrometer; a stable colloid will show no change ($\pm$1%) in optical density over 1 h.

The assay label also incorporates a functionalised dextran polymer passively adsorbed onto the surface of the carbon particle.

Dextran is a polymer of glucose and is composed of repeating units of $\alpha$-D-glucose-linked glucan (typically 95%) and 1,3-glucose residues as branches (typically 5%). Dextran is not subject to enzymatic degradation, unlike most other polysaccharides.

The polymer used in the present invention is a functionalised dextran, meaning that it contains additional functional groups to those otherwise present in dextran which can be used to form covalent bonds. Examples of functional groups include an amino group, a sulfate group, a ketone, an aldehyde, a carboxylic acid, a sulfonic acid, an activated carboxylic/sulfonic acid (such as an acid chloride or activated ester) and a thiol group. Some specific examples of commercially available functionalised dextrans are aminodextran, dextran sulfate, diethyl aminoethyl dextran and carboxymethyl dextran. A synthesis of aminodextrans is described in more detail in US 5,776,706. The functionalised dextran is preferably an aminodextran.

The functionalised dextran polymer of the present invention typically has a number average molecular weight of 3-2,000 KDa, preferably 5-100 KDa and most preferably 7-15 KDa. The molecular weight can be measured by gel permeation chromatography using commercially available dextran standards of known molecular weight (e.g. from Sigma Aldrich).

The functionalised dextran is attached to the carbon particle by non-covalent interactions. The functionalised dextran is passively adsorbed onto the surface of the carbon particle. Without wishing to be bound by theory, it is believed that the dextran is adsorbed into pores present on the surface of the carbon particle thereby displacing water molecules within the pores. The driving force for the reaction is probably entropic on account of the release of the bound water molecules.

The assay label also includes a first member of a complementary binding pair covalently bonded to the functionalised dextran.

In order to function in an assay, the label must contain part of a complementary binding pair. As previously explained, in an immunometric (also known as a sandwich or reagent-excess) immunoassay, one antibody raised to the analyte is located on the sensor and another antibody to the analyte is located on the carbon particles. The carbon particles then bind to the sensor in the presence of the analyte being detected. Analogous assays are possible with other macromolecules, such as proteins or nucleic acids. In this case, the first member of the complementary binding pair is a macromolecule selected from an antibody, a protein or a nucleic acid and the second member of the complementary binding pair is the analyte.

In a competition assay, there is either (a) an antibody on the carbon particles and an analogue of the analyte (i.e. a small molecule) on the sensor or (b) an analogue (i.e. a small molecule) on the carbon particles and an antibody on the sensor. Binding takes place in absence of analyte, and is perturbed (reduced) in the presence of analyte. In this case, the first member of the complementary binding pair may be a small molecule and the second member of the complementary binding pair is the antibody raised to the small molecule. Examples of the small molecule are therapeutic drugs (e.g. carbamazepine, cyclosporine, digoxin, theophylline and gentamycin), drugs of abuse (e.g. opiates, cocaine and amphetamine), vitamins (e.g. vitamin D, vitamin B12 and folate) and hormones (T3, T4, cortisol, progesterone, estradiol and testosterone).

The assay label of the present invention may be prepared by pre-treating the dextran polymer to form a dextran-small molecule conjugate. This conjugate can then be attached to the carbon particles to generate a stable conjugate. More than one small molecule can be attached to the dextran, so that the particle can be used in assays with controls or in multiplex assays.

The assay label of the present invention may also be prepared by attaching the dextran polymer to the carbon particles and separately, pre-treating an antibody, a protein or a nucleic acid with a reagent which will covalently bind to the functional groups on the dextran-coated particles. An example is to use aminodextran, which is reacted to add maleimide groups onto the surface of the dextran. The maleimido dextran is then coated onto the carbon particles. The antibody is introduced via a masked thiol, which can be unmasked to react with the maleimide group on the dextran. Masked thiols can be introduced by reaction with S-acetyl thioglycolic acid N-succinimide (SATA). In a preferred embodiment, the assay label further comprises a linker between the functionalised dextran and the complementary binding partner. For example, the maleimide groups can be introduced onto the dextran by addition of a heterobifunctional reagent, such as succinimidyl 4-(N-maleimidomethyl) cyclohexane-1-carboxylate (SMCC), or by analogues of SMCC, where the cyclohexane spacer is replaced by alternate spacers of longer length, such as polyalkylene glycol spacers. Following coupling of the SATA-activated antibody to the maleimido dextran carbon, it is advantageous to introduce a quench step to deactivate unreacted maleimide and thiol groups, usually by adding a maleimide quencher (e.g. mercaptoethanol) first, followed by a thiol quencher (e.g. N-ethyl maleimide, or a polyalkylene maleimide). These quenching reagents can then be removed by centrifugation and washing of the carbon colloid.

A useful guide for the sizes of the first member of the complementary binding pair are: the small molecule preferably has a molecular weight of 5,000 or less and the macromolecule (e.g. antibody, protein or nucleic acid) has a molecular weight above 5,0000, e.g. from 5,001-1,000,000.

An advantage of providing a linker is that it allows the macromolecule, e.g. the antibody, protein or nucleic acid, to form the correct tertiary structure by preventing steric interaction with the carbon particle and by permitting full solvation of the antibody, protein or nucleic acid. It has been found that this improves the signal:noise ratio by reducing non-specific binding in the assay. A preferred linker is polyethylene glycol, e.g. PEG12.

Accordingly, the present invention also provides a method for preparing an assay label comprising the following steps:

(i) providing an amorphous carbon particle, a functionalised dextran polymer and a first member of a complementary binding pair;
(ii) passively adsorbing the functionalised dextran polymer onto the surface of the amorphous carbon particle;
(iii) reacting the functionalised dextran polymer with the first member of the complementary binding pair to form a covalent bond between the functional groups on the functionalised dextran polymer and the first member of the complementary binding pair,

wherein steps (ii) and (iii) may be performed in either order, but follow step (i).

Preferably, the method further comprises reacting the functionalised dextran polymer with a bifunctional molecule prior to reaction with the first member of the complementary binding pair to form a linker between the functionalised dextran and the first member of the complementary binding pair. Other preferred features of the assay label described herein apply equally to this method.

The assay label of the present invention finds general applicability in the field of assays. However, the label is preferably used in the device described with reference to WO 2004/090512. Accordingly, the present invention also provides a device for performing an assay comprising:

> the assay label as described herein; a radiation source adapted to generate a series of pulses of electromagnetic radiation at a wavelength such that the absorption of the radiation by the label generates energy by non-radiative decay;
> a sample chamber containing a transducer having a pyroelectric or piezoelectric element and electrodes which is capable of transducing energy generated by non-radiative decay into an electrical signal; and a detector which is capable of detecting the electrical signal generated by the transducer.

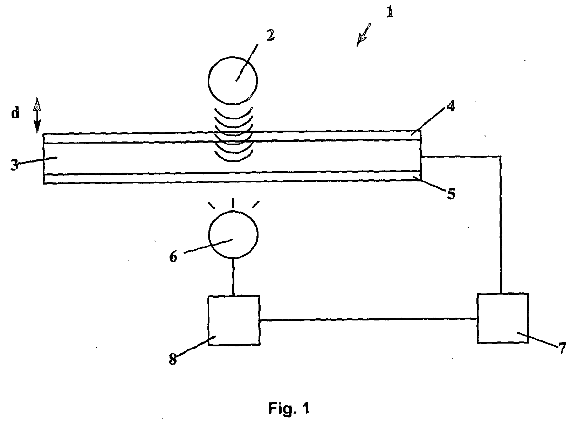

Fig. 1 shows a device 1 for use in accordance with the present invention which relies on heat generation in a particle 2 (herein, the carbon particle of the assay label) on irradiation of the particle 2 with electromagnetic radiation (the particle is shown above the transducer surface). For the sake of simplicity, only the particle is shown in Fig. 1 (the remaining components of the device will be described in further detail hereinbelow). Fig. 1 shows the device 1 in the presence of a particle 2. The device 1 comprises a pyroelectric or piezoelectric transducer 3 having electrode coatings 4,5. The transducer 3 is preferably a poled polyvinylidene fluoride film or a VDF-trifluoroethylene copolymer film. The electrode coatings 4,5 are preferably transparent and most preferably formed from indium tin oxide, although any transparent or semi-transparent electrode material would suffice, e.g. PEDOT (poly(3,4-ethylenedioxythiophene)). The electrodes preferably have a thickness of about 35 nm, although almost any thickness is possible from a lower limit of 1 nm below which the electrical conductivity is too low and an upper limit of 100 nm above which the optical transmission is too low (it should not be less than 80%T). In a particularly preferred embodiment, the transducer is an indium tin oxide-coated polyvinylidene fluoride film. An additional layer may be applied to the transducer 3, such as a parylene polymer layer, for passive adsorption of reagents to the sensor. A preferred embodiment is where the parylene layer is subsequently coated in a polymerised streptavidin layer.

The particle 2 is shown proximal to the transducer 3. An inherent feature of the carbon particles used in the present invention is that the particle 2 generates heat when irradiated by a source of electromagnetic radiation (typically termed "light") 6, preferably visible light. The light source may be, for example, an LED. The light source 6 illuminates the particle 2 with light of the appropriate wavelength. Although not wishing to be bound by theory, *it is* believed that the particle 2 absorbs the light to generate an excited state which then undergoes non-radiative decay thereby generating energy, indicated by the curved lines in Fig. 1. This energy is primarily in the form of heat (i.e. thermal motion in the environment) although other forms of energy, principally a shock wave, may also be generated. The energy is, however, detected by the transducer and converted into an electrical signal. The device is calibrated for the particular particle being measured and hence the precise form of the energy generated by the non-radiative decay does not need to be determined. Unless otherwise specified the term "heat" is used herein to mean the energy generated by non-radiative decay. The light source 6 is positioned so as to illuminate the particle 2. Preferably, the light source 6 is positioned opposite the transducer 3 and electrodes 4,5 and the particle 2 is illuminated through the transducer 3 and electrodes 4,5. The light source may be an internal light source within the transducer in which the light source is a guided wave system. The wave guide may be the transducer itself or the wave guide may be an additional layer attached to the transducer. The wavelength of illumination depends on the precise nature of the particle used.

The energy generated by the particle 2 is detected by the transducer 3 and converted into an electrical signal. The electrical signal is detected by a detector 7. The light source 6 and the detector 7 are both under the control of the controller 8. The light source 6 generates a series of pulses of light which is termed "chopped light". In principle, a single flash of light, i.e. one pulse of electromagnetic radiation, would suffice to generate a signal from the transducer 3. However, in order to obtain a reproducible signal, a plurality of flashes of light are used which in practice requires chopped light. The frequency at which the pulses of electromagnetic radiation are applied may be varied. At the lower limit, the time delay between the pulses must be sufficient for the time delay between each pulse and the generation of an electrical signal to be determined. At the upper limit, the time delay between each pulse must not be so large that the period taken to record the data becomes unreasonably extended. Preferably, the frequency of the pulses is from 1-50 Hz, more preferably 1-10 Hz and most preferably 2 Hz. This corresponds to a time delay between pulses of 20-1,000 ms, 100-1,000 ms and 500 ms, respectively. In addition, the so-called "mark-space" ratio, i.e. the ratio of on signal to off signal is preferably one although other ratios may be used without deleterious effect. There are some benefits to using a shorter on pulse with a longer off signal, in order to allow the system to approach thermal equilibrium before the next pulse perturbs the system. Sources of electromagnetic radiation which produce chopped light with different frequencies of chopping or different mark-space ratios are known in the art. The detector 7 determines the time delay between each pulse of light from light source 6 and the corresponding electrical signal detected by detector 7 from transducer 3. This time delay is a function of the distance, d. When particles are bound directly to the surface, the signal is preferably measured from 2-7 ms. For measuring particles through the depth of the chamber, longer time delays are used, e.g. 10-50 ms. The system can also be configured to measure the peak maximum in the signal, the time delay of which can change throughout the measurement process.

Any method for determining the time delay between each pulse of light and the corresponding electrical signal which provides reproducible results may be used.

It should be noted that the particle 2 may be separated from the transducer surface and that a signal may still be detected. Moreover, not only is the signal detectable through an intervening medium, but that different distances, d, may be distinguished (this has been termed "depth profiling") and that the intensity of the signal received is proportional to the concentration of the particle 2 at the particular distance, d, from the surface of the transducer 3. Moreover, it was found that the nature of the medium itself influences the time delay and the magnitude of the signal at a given time delay.

The purpose of the particle is to absorb the electromagnetic radiation generated by the radiation source to generate energy by non-radiative decay. The radiative decay is then converted to an electrical signal by the transducer. The wavelength of the electromagnetic radiation is such that the absorption of the radiation by the particles generates energy by non-radiative decay. The wavelength of the radiation is preferably 300-1,000 nm.

Red blood cells are present in a sample of blood, i.e. (unseparated) whole blood. These cells tend to sediment over time in a static system such as a test tube or container, since they are denser than the surrounding plasma in which they are dispersed. The system described in WO 2004/090512 is normally set up to minimise the signal from the red blood cells, by using a wavelength of light at which the signal from red blood cells is minimised (around 690 nm), and also by measuring the signal a few milliseconds after the light pulse, thus confining the output to heat generated in close proximity to the transducer.

The sample will typically be in the order of microlitres (e.g. 1-100 $\mu$L, preferably 1-30 $\mu$L). In order to hold a fluid sample, the transducer is preferably located in a chamber, the chamber having one or more side walls, an upper surface and a lower surface. Accordingly, the transducer is preferably located within a chamber for holding the sample in contact with the transducer. Preferably, the transducer is integral with the chamber, i.e. it forms one of the side walls, or upper or lower surface which define the chamber. In a preferred embodiment, the chamber has an upper surface and a lower surface and the transducer forms the upper surface. The sample may simply be retained by surface tension forces, for example, inside a capillary channel. The depth of the chamber is typically 50 $\mu$m to 1 cm, preferably 150-250 $\mu$m.

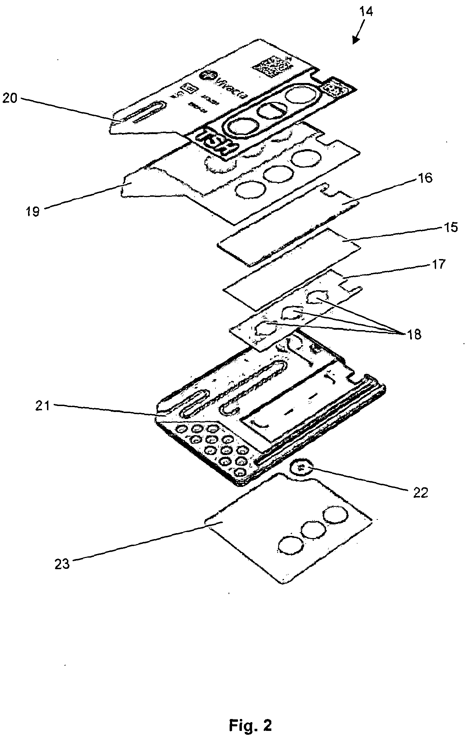

The device of the present invention may contain a plurality of chambers, preferably in fluid communication. The device preferably further contains an elongate sample collection passage having a sample collection end which is contact with the outside of the device and a sample delivery end which is in fluid communication with the sample chamber(s), as shown in the core 21 in Fig. 2. See WO 2011/027147 for further details.

In a preferred embodiment, the device further comprises an elongate sample collection passage having open ends and arranged to draw the fluid into the passage by capillary action, wherein the passage has a collection end and a delivery end and the delivery end is in fluid communication with the sample chamber. The passage may be provided along a first portion of its length with a region coated with an anticoagulant. This arrangement allows the sample to contact the anticoagulant to prevent clotting in the collection passage.

The device may take the form of a separate reader and cartridge, or an integrated device. In the former, the device is formed of a reader and a cartridge, in which the cartridge is releasably engageable with the reader, and in which the reader incorporates the radiation source and the detector, and the cartridge incorporates the transducer and the chamber. The reader is preferably a portable reader. The cartridge is preferably a disposable cartridge.

The present invention will now be described with reference to the following examples which are not intended to be limiting.

**Examples**

Example 1

*PVDF film sensor*

A poled piezo/pyroelectric polyvinylidene fluoride (PVDF) bimorph film, coated in indium tin oxide was used as the sensing device in the following examples. The indium tin oxide surface was coated with a layer of parylene (of approximate thickness 1 micron) by a vapour phase gas deposition process. This method involved the sublimation and subsequent pyrolysis of a paracyclophane precursor, followed by a free-radical polymerisation on the surface. See WO 2009/141637 for further details. The resulting film was then coated in polystreptavidin solution (200 $\mu$g/mL in PBS - 10 mmol/L phosphate buffer containing 2.7 mmol/L KCl, 137 mmol/L NaCl and 0.05% Tween) by incubation at room temperature overnight. Polystreptavidin was prepared as described by Tischer et al (US 5,061,640). The polystreptavidin provides a universal binding sensor to which other molecules can be attached through the high affinity biotin-streptavidin reaction.

## Example 2

*Preparation of the cartridge*

As shown in Fig. 2, a cartridge 14 was fabricated to perform the measurement. The cartridge 14 was fabricated from a piezo/pyrofilm 15 supported on a stiffener 16. A pressure sensitive adhesive-coated polyester film 17 die-cut to form three sample chambers 18 was applied to the surface. Provision was made to allow for electrical connections to the top and bottom surfaces of the piezo/pyrofilm 15 in order to detect the charge generated. The cartridge 14 was then formed by sandwiching the above components between a top cover 19, to which a label 20 was applied, and a core 21, seal 22 and bottom cover 23.

Measurements were carried out by charging the sample chambers with the sample. The piezo/pyrofilm 15 was irradiated through the holes in the top cover 19 with chopped LED light sequentially with LEDs. For each LED pulse, a voltage is measured across the piezo/pyrofilm 15 using an amplifier and analogue to digital (ADC) converter. The time-resolved ADC signal is plotted over time.

## Examples 3-13

In the examples that follow, assays are carried out using positive and negative controls to improve the accuracy and precision of the measurement. Of the two controls, one defines the maximum binding rate expected under diffusion control and one defines the minimum signal expected in the absence of signal (normally slightly negative owing to particle sedimentation). The signal output is defined ratiometrically by these two controls.

## Example 3

*Preparation of a carbon anti-cTnI colloid by passive adsorption (carbon conjugate 1)*

400 μg of anti-cardiac troponin I mouse monoclonal antibody clone 560 (Hytest, ,Finland) was prepared in 2 mL of 10 mM potassium phosphate buffer, pH 7.2. To this was added 1 mL of a 0.2% w/v suspension of SB4 carbon particles (Degussa) in water. The mixture was left stirring at R/T for 2 h, then diluted by addition of 6 ml of 10 mM potassium phosphate buffer containing 4.5% sucrose, 0.15% BSA and 0.075% PEG 20k (wash and storage buffer). This solution was then purified by three cycles of centrifugation, pelleting, washing, sonication and re-suspension in the storage buffer. Finally, 4.5 μL of Proclin 950 preservative was added and the solution was stored at 4°C.

## Example 4

*Preparation of a carbon dextran anti-cTnI colloid by primary reaction with a dextran reagent followed by covalent attachment of anti cTnI antibody (carbon conjugate 2)*

10 KDa aminodextran (Invitrogen) was dissolved at 5 mg/mL in 0.1 M phosphate pH 7.5 buffer, and 11.6 mL of this solution was incubated for 60 mins at 20°C with 3300 μL of a 30 mg/mL solution (in DMSO) of N-hydroxysuccinimide-PEG12-maleimide (Thermo Fisher), equivalent to a 20:1 molar excess over aminodextran. After 60 mins the reaction was quenched with glycine (86.73 mg) and then purified on a Sephadex G50 column, eluting with 0.1 M phosphate pH 7.5 buffer. The product peak was analysed with the phenol sulfuric acid assay to determine the concentration as 1.776 mg/mL. The aminodextran maleimide was stored at -80°C until required.

100 mg of SB4 carbon particles (Degussa) in water (50 mL) were sonicated for 0.5 h. From the resulting suspension 1.667 mL of carbon colloid was mixed with aminodextran-maleimide solution (2.252 mL) and 0.1 M phosphate pH 7.5 buffer (8.859 mL). The mixture was sonicated for 30 s then roller mixed for 90 mins. The mixture then was centrifuged and pelleted for 15 mins and the supernatant was discarded. The mixture was resolubilised by sonication in 0.01 M phosphate buffer pH 7.2 (10 mL) for 30 s and the suspension was again spun down. A total of three wash cycles were carried out.

In parallel, 11.66 mg of anti-troponin antibody, clone 560 (HyTest) was desalted into phosphate buffer using a single PD-10 Sephadex G25M disposable column and then activated with 5 equivalents of N-succinimidyl-S-acetyl thioacetate (SATA) (Thermo-Fisher) (90 μL of a 1 mg/mL solution in DMSO) at 20°C for 1 h, then deprotected using hydroxylamine buffer (315 μL) for 15 min. The thiolated antibody was purified on 2 PD-10 Sephadex G25M disposable columns into 0.1 M phosphate pH 7.5 buffer giving a solution containing 1.76 mg/mL of antibody. The thiol incorporation level was measured as 1.9 thiols per antibody using the Ellman's assay.

After the final wash cycle of the carbon coated in aminodextran maleimide, the supernatant was discarded and the thiolated antibody (10 mg, 5.682 mL) was added along with 4.318 mL of 0.1 M phosphate buffer pH 7.5. The mixture was resolubilised by sonication for 30 s then the reaction was roller-mixed for 1 h 45 mins before being quenched with 2-mercaptoethanol (200 μL, 1mg/mL in water) for 15 mins, then quenched with PEG12-maleimide (Thermo Fisher) (180 μL, 10mg/mL) for a further 15 mins. The mixture was then spun down as before and underwent a further three wash cycles before a final resolubilisation in 0.01 M phosphate + 0.1% BSA + 3% sucrose + 0.05% PEG 20K pH 7.2 buffer (375

μL) with 60 s of sonication.

Similarly, a carbon conjugate was prepared under exactly the same conditions as above, except that the antibody was not activated with SATA. All preparation and wash steps were carried out in a similar manner.

Example 5

*Comparison of the binding capacities of two different carbon anti-cTnI colloids*

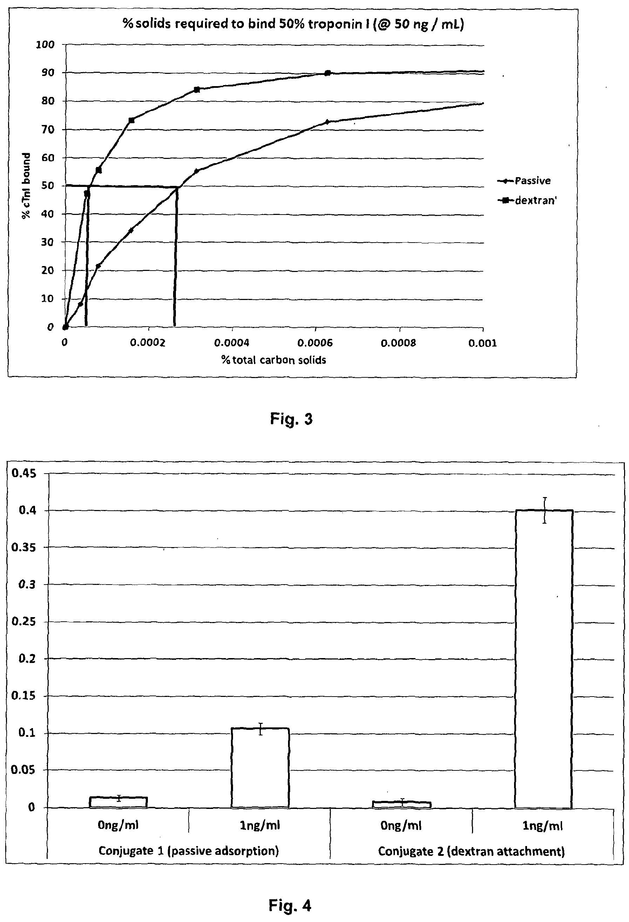

The anti-cTnI carbon conjugates from Examples 3 and 4 were diluted into solutions of cardiac troponin I in phosphate buffered saline plus 0.5% bovine serum albumin. The final concentration of troponin was 50 ng/mL and the final concentration of carbon solids ranged from 0.000039% w/v to 0.0035% w/v. A control was also run with no carbon conjugate added. Total volume per reaction was 500 μL. The carbon solids concentration was checked by measuring the optical density of the solution at 450 nm and comparing against a standard curve prepared with SB4 carbon suspension in water. After incubation for 30 mins, the carbon solids were removed from each reaction mixture by centrifugation. 50 μL of each mixture was then diluted into 450 μL of troponin-free serum and the concentration of unbound troponin in each sample was measured on an Abbott Architect Clinical Immunoassay analyser. The quantity of bound troponin at each concentration of carbon solids was calculated by subtraction, this was then plotted and the total carbon conjugate required to bind 50% of the troponin for each of the two conjugates was interpolated from the data (shown in Fig. 3).

The concentration of bound troponin is governed by the equilibrium equation:

$$K_a = \frac{[Ab * Ag]}{[Ab][Ag]}$$

where [Ab*Ag] is the concentration of troponin-antibody complex, [Ag] is the concentration of free troponin and [Ab] is equal to the concentration of antibody in the reaction mixture with available binding sites. At 50% binding, the concentration of free and complexed troponin is equal, so the equation for the equilibrium (law of mass action) simplifies from to $K_a = 1/[Ab]$. Since the same antibody is used for each conjugate, the percentage of carbon solids required to achieve 50% binding gives a direct measure of the relative quantity of active antibody for the two conjugates. It can be observed that around five times more of the passively adsorbed conjugate (0.00025% solids) is required to bind 50% of the troponin compared to the dextran conjugate (0.00005% solids), indicating that there is around five times more active antibody on the dextran conjugate.

Example 6

*Imm unoassay for cTnI using carbon colloid passively coated with antibody*

An immunoassay for troponin I was carried out using the carbon conjugate prepared in Example 3. The assay was carried out using the pyroelectric detector system described in WO 2004/090512, utilising controls, as described hereinabove. In summary, three separate areas of a PVDF sensor were coated in three different antibodies, the first a non-specific negative control antibody, the second a monoclonal antibody directed against troponin (Hytest clone 19C7) and the third a polyclonal goat anti-mouse antibody. The antibodies had previously been biotinylated, and they were coated onto a universal polymerised streptavidin surface which had previously been coated onto the PVDF sensor. The sensor was enclosed in a fluidic device fabricated from an injection moulded part and a number of die-cut pressure-sensitive adhesives, as shown in Fig. 2, which generates three separate, interconnected chambers for three separate measurements. Human plasma samples mixed with carbon conjugate 1 and buffer (35 mM HEPES, 42 mM EGTA, 280mM NaCl, 1.22% Tween) were prepared, either with undetectable troponin levels, or spiked with human troponin ITC complex to around 1 ng/mL. The carbon conjugate was diluted 1:12, to give a final carbon concentration of approximately 0.004% w/v. The three areas of the sensor surface were exposed to the reaction mixture and were illuminated sequentially using high-powered LEDs (690 nm). The pyroelectric signal generated at each area was amplified and monitored over the course of 10 mins. The rate of change of signal in each chamber over the 10 mins was then calculated, and the rate of binding of carbon particles to the anti-troponin antibody at area 2 was calculated relative to the other two areas. Thus the kinetic signal in chamber 3 was defined as 1.00, the kinetic signal in chamber 1 was defined as 0.00, and the assay output is where the kinetic signal in chamber 2 lies between the other 2 chambers. If there is sufficient antibody on the carbon particles, then all of the particles will bind to the goat anti-mouse surface in chamber 3, and the rate of binding will be governed solely by diffusion kinetics. Ten repeats were carried out at each concentration, to generate a mean signal and a standard deviation on the measurement.

The data are shown in Fig. 4, with 1 SD error bars. The signal in the presence of 1 ng/mL troponin I was approximately 0.11, i.e. binding took place at 11% of the maximum diffusion rate. The magnitude of the signal and the observed imprecision gave an analytical sensitivity of around 120 pg/mL.

## Example 7

*Immunoassay for cTnI using carbon colloid actively coated with antibody*

An immunoassay for troponin was carried out using the carbon conjugate prepared in Example 4. The only difference between this experiment and that described in Example 6 was that the carbon conjugate was different, all other assay conditions were identical. In this instance, the observed signal (*see* Fig. 4) was almost four times higher, but the imprecision at the zero-analyte level remained relatively unchanged. This improvement in signal-to-noise reduced the analytical sensitivity to 35 pg/mL. It was noted that the signals in chambers 1 and 3 were similar in both Examples 6 and 7, the main change was in chamber 2. It was concluded that the higher level of active antibody on conjugate 2 led to more troponin being bound to the particle, and hence an increase in the rate of binding of the particles to the sensor surface in chamber 2. A control experiment was also carried out using the conjugate from Example 4 that had been prepared without SATA activation of the antibody. This gave no distinguishable signal in chambers 2 or 3, indicating that the antibody does not bind to the maleimido dextran unless the antibody has free thiol groups available to form a covalent bond.

## Example 8

*Preparation of co-conjugate of bovine serum albumin with fluorescein and digoxigenin*

Bovine serum albumin (BSA, Sigma) was dissolved at 15 mg/mL in 0.1 M sodium hydrogen carbonate solution, and 2.267 mL (34 mg BSA) of this solution was incubated for 60 mins at 20°C with 251 mL of a 20 mg/mL solution (in DMSO) of digoxigenin N-hydroxysuccinimide ester.(Roche). After 60 mins, 518 μL (7 mg) of this solution was removed and 61 μL of a 15 mg/mL solution (in DMSO) of fluorescein N-hydroxysuccinimide ester (Perbio) added to this aliquot. This solution was incubated for a further 60 mins at 20°C. The calculated molar ratios of digoxigenin:BSA and fluorescein:BSA were 20:1 and 15:1, respectively.

The crude BSA-digoxigenin-fluorescein product was purified into PBS pH 7.1 buffer containing 0.1% sodium azide on a single PD-10 Sephadex G25M column (GE), yielding 1.6 mL of a solution containing approximately 4.30 mg/mL of BSA, which was filtered to 0.2 μm (Minisart filter, Sartorius).

## Example 9

*Preparation of co-conjugate of amino dextran with fluorescein and digoxigenin*

70 KDa aminodextran (Invitrogen) was dissolved at 5.0 mg/mL in 0.1 M sodium hydrogen carbonate solution, and 1.0 mL (5mg aminodextran) of this solution was incubated for 60 mins at 20°C simultaneously with 19 μL of a 5.0 mg/mL solution (in DMSO) of digoxigenin N-hydroxysuccinimide ester (Roche) and 14 μL of a 5.0 mg/mL solution (in DMSO) of fluorescein N-hydroxysuccinimide ester (Perbio), both reagents being at a 2:1 molar excess over aminodextran

The crude aminodextran-digoxigenin-fluorescein product was purified into PBS pH 7.1 buffer containing 0.1% sodium azide on a single PD-10 Sephadex G25M column (GE), yielding 2.7 mL of a solution containing approximately 1.67 mg/mL of aminodextran. Proclin 950 (27 μL) was added and the solution filtered to 0.2 μm (Minisart filter, Sartorius).

## Example 10

*Preparation of carbon bovine serum albumin digoxigenin/fluorescein co-conjugate*

The bovine serum albumin conjugate from Example 8 was diluted to 150 μg/mL in 10 mM phosphate buffer, then 1 mL of this solution was added 0.5 mL of a 0.2% solution of SB4 carbon in deionised water. After 2 h, 3 mL of the wash/storage buffer from Example 3 was added, then the mixture was centrifuged, pelleted, washed and re-suspended (in the same buffer), then finally re-constituted in 2.5 mL of the storage buffer and kept at 4°C.

## Example 11

*Preparation of carbon dextran digoxigenin/fluorescein co-conjugate*

The dextran conjugate from Example 9 was diluted to 25 μg/mL in 10 mM phosphate buffer, then 1 mL of this solution was added 0.5 mL of a 0.2% solution of SB4 carbon in deionised water. After 2 h, 3 mL of the wash/storage buffer from Example 3 was added, then the mixture was centrifuged, pelleted, washed and re-suspended (in the same buffer), then finally re-constituted in 2.5 mL of the storage buffer and kept at 4°C.

## Example 12

*Assay performance and stability of digoxigenin/fluorescein BSA carbon*

A competitive assay for digoxin was carried out using the carbon conjugate prepared in Example 10. The assay was carried out using the pyroelectric detector system described in WO 2004/090512, utilising controls, as described hereinabove. In summary, three separate areas of a PVDF sensor were coated in three different antibodies, the first a non-specific negative control antibody, the second a monoclonal antibody directed against digoxin (Jackson Immunoresearch clone HY-A.1) and the third a monoclonal anti-FITC antibody. The antibodies had previously been biotinylated, and they were coated onto a universal polymerised streptavidin surface which had previously been coated onto the PVDF sensor. The sensor was enclosed in a fluidic device fabricated from an injection moulded part and a number of die-cut pressure-sensitive adhesives, as shown in Fig. 2, which generates three separate, interconnected chambers for three separate measurements. Human plasma samples mixed with carbon conjugate 8 and buffer (66 mM Tris, 14 mM $MgCl_2$ and 0.05% Tween 20) were prepared, either with undetectable digoxin levels, or spiked with purified digoxin to around 5 ng/mL. The carbon conjugate was diluted 1:14 in the sample, to give a final carbon concentration of approximately 0.0035% w/v. The three areas of the sensor surface were exposed to the reaction mixture and the three areas were illuminated sequentially using high-powered LEDs (690 nm). The pyroelectric signal generated at each area was amplified and monitored over the course of 10 mins. The rate of change of signal in each chamber over the 10 mins was then calculated, and the rate of binding of carbon particles to the anti-digoxin antibody in chamber 2 was calculated relative to the other two chambers. Ten repeats were carried out at each concentration to generate a mean signal and a standard deviation on the measurement. In this example, a high signal is expected in chamber 2 in the absence of digoxin, since the antibody on the surface recognises the digoxigenin on the surface of the carbon particle. If digoxin is present in the sample, then this perturbs the binding in chamber 2 by blocking antibody binding sites on the sensor surface. The assay was repeated over a number of days, with the sample being prepared fresh each time, using carbon conjugate 8 which was stored in liquid format at 4°C.

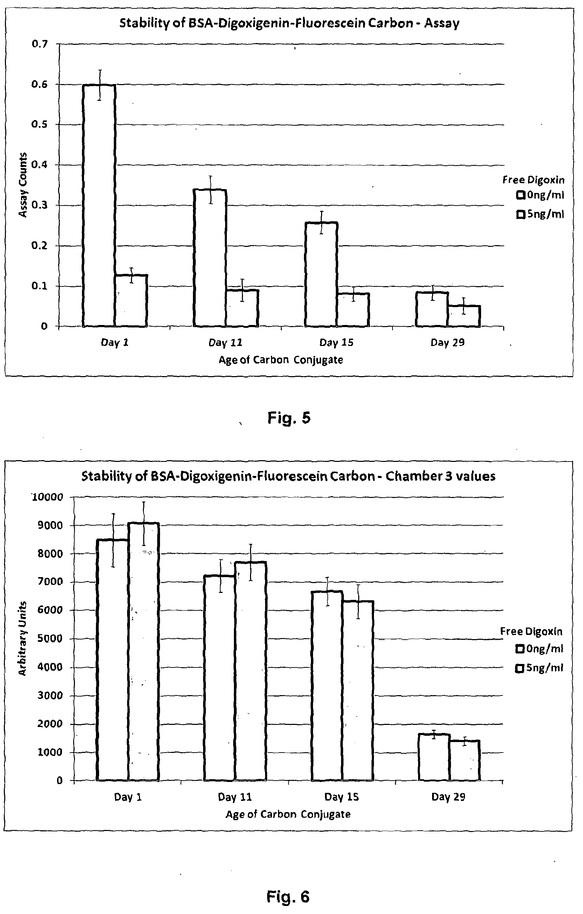

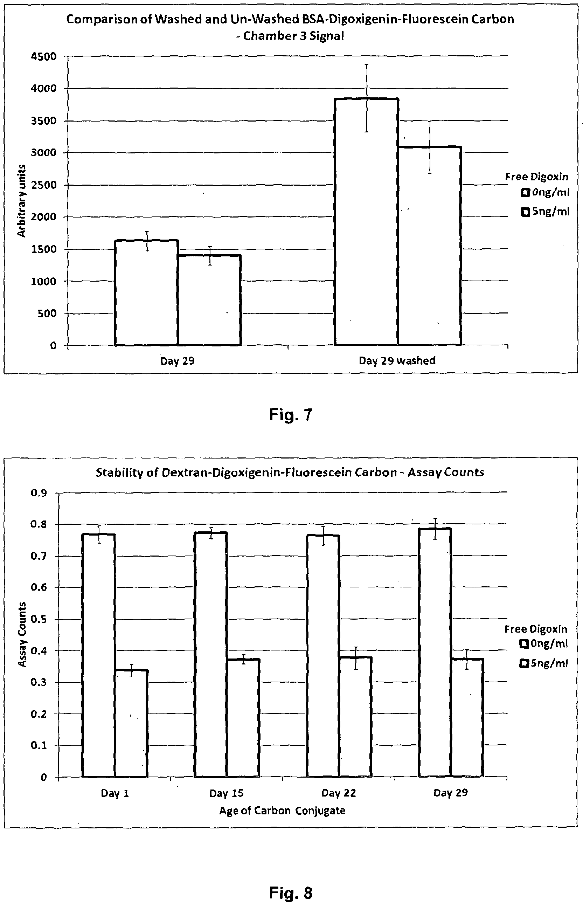

The data are shown in Fig. 5, with 1 SD error bars. On day zero the assay performs as expected, giving a high signal in the absence of digoxin, and a low signal in the presence of digoxin. However, it can be seen that the assay performance is changing over time, with the signal in the absence of digoxin reducing upon storage of the carbon conjugate. The signal in chamber 1 of these assays (the minimum binding control) remains largely unchanged over time, it is the signal in chambers 2 (anti-digoxin) and 3 (anti-FITC) which is changing, with the signal dropping over time in both chambers. Fig. 6 shows the binding rate in chamber 3 over time. The signal in chamber 3 is independent of the digoxin concentration, since the binding is to the fluorescein group on the carbon particle. The drop in signal would suggest that the BSA-digoxigenin-FITC conjugate is desorbing from the surface of the carbon particle over time upon storage. Any unbound BSA-digoxigenin-FITC in solution would compete for the binding sites on the surface and lower the rate of binding. This was confirmed by centrifugation, pelleting, washing and resuspension of the carbon conjugate. This led to an increase in binding in chamber 3, as shown in Fig. 7.

Example 13

*Assay performance and stability of digoxigenin/fluorescein dextran carbon*

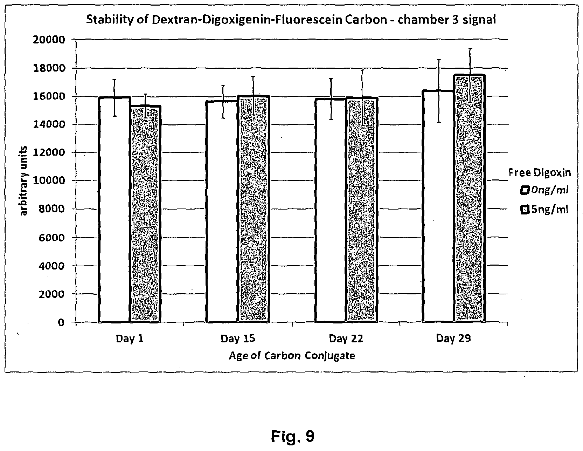

A competitive assay for digoxin was carried out using the carbon conjugate prepared in Example 11. The reaction conditions were identical to those used in Example 12, except for the carbon conjugate. The ratiometric assay counts are shown in Fig. 8. It is observed that the final assay signal (ratiometric output) shows no change over the 29 days that the carbon was stored at 4°C. The chamber 3 values are shown in Fig. 9, confirming that there was no deterioration in the conjugate over this period.

**Claims**

1. An assay label comprising an amorphous carbon particle, a functionalised dextran polymer passively adsorbed onto the surface of the carbon particle and a first member of a complementary binding pair covalently bonded to the functionalised dextran polymer.

2. An assay label as claimed in claim 1, wherein the carbon particle has a particle size of 25-250 nm.

3. An assay label as claimed in claims 1 or 2, wherein the functionalised dextran has a number average molecular weight of 3-2,000 KDa.

4. An assay label as claimed in any preceding claim, wherein the functionalised dextran contains at least one functional group selected from a sulfate group, a ketone, an aldehyde, a carboxylic acid, a sulfonic acid, an activated carboxylic/sulfonic acid and a thiol group.

5. An assay label as claimed in claim 4, wherein the functionalised dextran is an aminodextran.

6. An assay label as claimed in any preceding claim, wherein the first member of the complementary binding pair is a small molecule.

7. An assay label as claimed in any of claims 1 to 5, wherein the first member of the complementary binding pair is selected from an antibody, a protein and a nucleic acid.

8. An assay label as claimed in claim 7, wherein the assay label further comprises a linker between the functionalised dextran and the first member of the complementary binding pair.

9. A method for preparing an assay label comprising the following steps:

(i) providing an amorphous carbon particle, a functionalised dextran polymer and a first member of a complementary binding pair;
(ii) passively adsorbing the functionalised dextran polymer onto the surface of the amorphous carbon particle;
(iii) reacting the functionalised dextran polymer with the first member of the complementary binding pair to form a covalent bond between the functional groups on the functionalised dextran polymer and the first member of the complementary binding pair,

wherein steps (ii) and (iii) may be performed in either order, but follow step (i).

10. The method as claimed in claim 9, further comprising reacting the functionalised dextran polymer with a bifunctional molecule prior to reaction with the first member of the complementary binding pair to form a linker between the functionalised dextran and the first member of the complementary binding pair.

11. A device for performing an assay comprising:

an assay label as claimed in any of claims 1 to 8;
a radiation source adapted to generate a series of pulses of electromagnetic radiation at a wavelength such that the absorption of the radiation by the label generates energy by non-radiative decay;
a sample chamber containing a transducer having a pyroelectric or piezoelectric element and electrodes which is capable of transducing energy generated by non-radiative decay into an electrical signal; and
a detector which is capable of detecting the electrical signal generated by the transducer.

12. A device as claimed in claim 11 wherein the chamber has an upper surface and a lower surface and the transducer forms the upper surface.

13. A device as claimed in claim 11 or 12, wherein the device is formed of a reader and a cartridge, in which the cartridge is releasably engageable with the reader, and in which the reader incorporates the radiation source and the detector, and the cartridge incorporates the label, the transducer and the chamber.

**Patentansprüche**

1. Testetikett, umfassend ein amorphes Kohlenstoffteilchen, ein funktionalisiertes Dextranpolymer, das passiv auf der Oberfläche des Kohlenstoffteilchens adsorbiert ist, und ein erstes Element eines komplementären Bindungspaares, das kovalent an das funktionalisierte Dextranpolymer gebunden ist.

2. Testetikett nach Anspruch 1, wobei das Kohlenstoffteilchen eine Teilchengröße zwischen 25 und 250 nm aufweist.

3. Testetikett nach Anspruch 1 oder 2, wobei das funktionalisierte Dextran ein Zahlenmittel des Molekulargewichts von 3-2.000 KDa aufweist.

4. Testetikett nach einem der vorstehenden Ansprüche, wobei das funktionalisierte Dextran wenigstens eine funktionelle Gruppe aufweist, die ausgewählt ist aus einer Sulfatgruppe, einem Keton, einem Aldehyd, einer Carbonsäure, einer Sulfonsäure, einer aktivierten Carbon-/Sulfonsäure und einer Thiolgruppe.

5. Testetikett nach Anspruch 4, wobei das funktionalisierte Dextran ein Aminodextran ist.

6. Testetikett nach einem der vorstehenden Ansprüche, wobei das erste Element des komplementären Bindungspaares ein kleines Molekül ist.

7. Testetikett nach einem der Ansprüche 1 bis 5, wobei das erste Element des komplementären Bindungspaares ausgewählt ist aus einem Antikörper, einem Protein und einer Nukleinsäure.

8. Testetikett nach Anspruch 7, wobei das Testetikett ferner einen Linker zwischen dem funktionalisiertem Dextran und dem ersten Element des komplementären Bindungspaares umfasst.

9. Verfahren zum Herstellen eines Testetiketts, umfassend die folgenden Schritte:

(i) Bereitstellen eines amorphen Kohlenstoffteilchens, eines funktionalisierten Dextranpolymers und eines ersten Elements eines komplementären Bindungspaares;
(ii) passives Adsorbieren des funktionalisierten Dextranpolymers auf der Oberfläche des amorphen Kohlenstoffteilchens
(iii) Reagieren des funktionalisierten Dextranpolymers mit dem ersten Element des komplementären Bindungspaares, um eine kovalente Bindung zwischen den funktionellen Gruppen an dem funktionalisiertem Dextranpolymer und dem ersten Element des komplementären Bindungspaares zu bilden,

wobei die Schritte (ii) und (iii) in beliebiger Reihenfolge, jedoch nach Schritt (i) ausgeführt werden können.

**10.** Verfahren nach Anspruch 9, ferner umfassend das Reagieren des funktionalisierten Dextranpolymers mit einem bifunktionalen Molekül vor der Reaktion mit dem ersten Element des komplementären Bindungspaares, um einen Linker zwischen dem funktionalisierten Dextran und dem ersten Element des komplementären Bindungspaares zu bilden.

**11.** Vorrichtung zur Durchführung eines Tests, umfassend:

ein Testetikett nach einem der Ansprüche 1 bis 8;
eine Strahlungsquelle, die eine Reihe von Impulsen elektromagnetischer Strahlung mit einer Wellenlänge erzeugen kann, so dass die Absorption der Strahlung durch das Etikett durch nichtstrahlenden Zerfall erzeugt;
eine Probenkammer, die einen Messwandler mit einem pyroelektrischen oder piezoelektrischen Element und Elektroden enthält, der durch nichtstrahlenden Zerfall erzeugte Energie in ein elektrisches Signal umwandeln kann; und einen Detektor, der das durch den Messwandler erzeugte elektrische Signal erkennen kann.

**12.** Vorrichtung nach Anspruch 11, wobei die Kammer eine obere Oberfläche und eine untere Oberfläche aufweist, und wobei der Messwandler die obere Oberfläche bildet.

**13.** Vorrichtung nach Anspruch 11 oder 12, wobei die Vorrichtung aus einem Leser und einer Patrone gebildet wird, wobei die Patrone trennbar mit dem Leser eingreifen kann, und wobei der Leser die Strahlungsquelle und den Detektor inkorporiert, und wobei die Patrone das Etikett, den Messwandler und die Kammer inkorporiert.

**Revendications**

**1.** Marque de dosage comprenant une particule de carbone amorphe, un polymère de dextran fonctionnalisé passivement adsorbé sur la surface de la particule de carbone et un premier élément d'une paire de liaison complémentaire liée par covalence au polymère de dextran fonctionnalisé.

**2.** Marque de dosage selon la revendication 1, la particule de carbone ayant une taille de particule comprise entre 25 et 250 nm.

**3.** Marque de dosage selon la revendication 1 ou 2, le dextran fonctionnalisé ayant une masse moléculaire moyenne en nombre comprise entre 3 et 2 000 KDa.

**4.** Marque de dosage selon l'une quelconque des revendications précédentes, le dextran fonctionnalisé contenant au moins un groupe fonctionnel choisi parmi un groupe sulfate, une cétone, un aldéhyde, un acide carboxylique, un acide sulfonique, un acide carboxylique/sulfonique activé et un groupe thiol.

**5.** Marque de dosage selon la revendication 4, le dextran fonctionnalisé étant un aminodextran.

**6.** Marque de dosage selon l'une quelconque des revendications précédentes, le premier élément de la paire de liaison complémentaire étant une petite molécule.

**7.** Marque de dosage selon l'une quelconque des revendications 1 à 5, le premier élément de la paire de liaison complémentaire étant choisi parmi un anticorps, une protéine et un acide nucléique.

**8.** Marque de dosage selon la revendication 7, la marque de dosage comprenant en outre un lieur entre le dextran fonctionnalisé et le premier élément de la paire de liaison complémentaire.

**9.** Procédé de préparation d'une marque de dosage comprenant les étapes consistant à :

(i) fournir une particule de carbone amorphe, un polymère de dextran fonctionnalisé et un premier élément d'une paire de liaison complémentaire ;
(ii) passivement adsorber le polymère de dextran fonctionnalisé sur la surface de la particule de carbone amorphe ;
(iii) faire réagir le polymère de dextran fonctionnalisé avec le premier élément de la paire de liaison complémentaire pour former une liaison covalente entre les groupes fonctionnels sur le polymère de dextran fonctionnalisé et le premier élément de la paire de liaison complémentaire,

les étapes (ii) et (iii) pouvant être effectuées dans n'importe quel ordre, mais après l'étape (i).

**10.** Procédé selon la revendication 9, comprenant en outre l'étape consistant à faire réagir le polymère de dextran fonctionnalisé avec une molécule bifonctionnelle avant la réaction avec le premier élément de la paire de liaison complémentaire pour former un lieur entre le dextran fonctionnalisé et le premier élément de la paire de liaison complémentaire.

**11.** Dispositif pour effectuer un dosage comprenant :

une marque de dosage selon l'une quelconque des revendications 1 à 8 ;
une source de rayonnement conçue pour générer une série d'impulsions de rayonnement électromagnétique à une longueur d'onde telle que l'absorption du rayonnement par la marque génère de l'énergie par désintégration non rayonnante ;

une enceinte d'échantillonnage contenant un transducteur ayant un élément pyroélectrique ou piézoélectrique et des électrodes pouvant transducter de l'énergie générée par désintégration non rayonnante en un signal électrique ; et

un détecteur apte à détecter le signal électrique généré par le transducteur.

**12.** Dispositif selon la revendication 11, la chambre ayant une surface supérieure et une surface inférieure et le transducteur formant la surface supérieure.

**13.** Dispositif selon la revendication 11 ou 12, le dispositif étant formé d'un lecteur et d'une cartouche, la cartouche venant en prise de manière libérable avec le lecteur, et le lecteur incorporant la source de rayonnement et le détecteur, et la cartouche incorpotant la marque, le transducteur et la chambre.

**Fig. 1**

**Fig. 2**

Fig. 3

Fig. 4

**Fig. 5**

**Fig. 6**

**Fig. 7**

**Fig. 8**

**Fig. 9**

**REFERENCES CITED IN THE DESCRIPTION**

**Patent documents cited in the description**

- WO 2004090512 A
- US 3442819 A
- US 4028465 A
- US 4760030 A
- US 5252496 A
- US 5559041 A
- US 6506612 B
- US 5529901 A
- US 5641689 A
- WO 2005121163 A
- KR 20110128606
- US 5776706 A
- WO 2011027147 A
- WO 2009141637 A
- US 5061640 A, Tischer

**Non-patent literature cited in the description**

- **N. POZNANSKI ; W.J. POZNANSKI.** *Clin. Chem.,* 1969, vol. 15, 908-918